(19)

Europäisches
Patentamt

European
Patent Office

Office européen
des brevets

(11) **EP 2 050 743 A1**

(12) **EUROPEAN PATENT APPLICATION**

(43) Date of publication:
 **22.04.2009 Bulletin 2009/17**

(51) Int Cl.:
 ***C07D 311/72*** *(2006.01)*    ***C01B 11/24*** *(2006.01)*
 ***C01F 5/28*** *(2006.01)*    ***C01F 7/50*** *(2006.01)*

(21) Application number: **07020498.7**

(22) Date of filing: **19.10.2007**

(84) Designated Contracting States:
 **AT BE BG CH CY CZ DE DK EE ES FI FR GB GR
 HU IE IS IT LI LT LU LV MC MT NL PL PT RO SE
 SI SK TR**
 Designated Extension States:
 **AL BA HR MK RS**

(71) Applicant: **Humboldt-Universität zu Berlin
 D-10099 Berlin (DE)**

(72) Inventors:
 • **Kemnitz, Erhard
 10405 Berlin (DE)**

 • **Coman, Simona
 Bucharest (RO)**
 • **Rüdiger, Stephan
 15834 Rangsdorf (DE)**
 • **Wuttke, Stefan
 12099 Berlin (DE)**

(74) Representative: **Bohmann, Armin K.
 Bohmann & Loosen
 Anwaltssozietät
 Nymphenburger Strasse 1
 80335 München (DE)**

(54) **Method for the synthesis of dl.(alpha)-tocopherol and means therefore**

(57) The present invention is related to a method for synthesizing dl-alpha-tocopherol comprising the steps of
- providing 2,3,5-trimethylhydroquinone;
- providing isophytol;

- providing a solid fluoride catalyst; and
- reacting the 2,3,5-trimethylhydroquinone with the isophytol in the presence of the catalyst.

**EP 2 050 743 A1**

**Description**

The present invention is related to alkylation-condensation reactions and means therefore. More particularly, the present invention is related to the synthesis of *dl*-[α]-tocopherol and a catalyst useful for such synthesis.

By Vitamin E is termed a group of related fat-soluble tocochromanols, including eight naturally occurring components, which exhibit antioxidant activity and are nutritionally essential. Among these components α-tocopherol is chemically and biologically the most active [Catal. Today, 121 (2007) 45; Mol. Nutr. Food Res., 49 (2005) 7]. More recently was demonstrated that besides its antioxidant properties α-tocopherol shows promising results in the prevention and treatment of heart disease, cancer and Alzheimer's disease [Biomedicine & Pharmacotherapy 59 (2005) 380; Nutrition Research 25 (2005) 877]. The main market of Vitamin E is feed market (73 %) followed by pharmaceutical (24 %), cosmetics and food market (3 %) and the demand is constantly increasing [Catal. Today, 121 (2007) 45]. Numerous processes have therefore been developed for its synthesis.

The classical industrial synthesis of (all-rac)-[α]-tocopherol, made via the acid-catalysed condensation of 2,3,6-trimethylhydroquinone (TMHQ) with isophytol (IP), faces many problems caused by corrosion from the acidic media, contamination of the waste water with acids and zinc ions and the difficult purification of (all-rac)-[α]-tocopherol via distillation under high vacuum at 200 °C [Chimica, 50 (1996) 563]. Part of these problems is due to the use of homogenous catalysts as $ZnCl_2$/HCl [U.S. Pat. No. 2,411,969 of Hoffmann-La Roche, U. S. Pat. No. 4,239,691 and DE 3 203 487 of BASF, U. S. Pat. No. 6,005,122 of BASF; DE 27 43 920]; $BF_3$, $AlCl_3$ or $FeCl_2$/Fe/HCl, in various organic solvents.

It is common to all these known processes that the vitamin E cannot be prepared in the required purity on the industrial scale.

To overcome this problem, the reaction disclosed in DE 27 43 920 is carried out in the presence of a mixture of silica and alumina or silica gel and zinc chloride and a strong acid such as concentrated $HCl_2$, $H_2SO_4$ $H_3PO_4$ or p-toluenesulfonic acid.

The disadvantages of this process are, as with many of the above mentioned processes, that problems with corrosion arise, and that the waste water may be polluted with zinc ions.

Because of these difficulties other acid catalysts have been described for the condensation of TMHQ and IP. Thus, for example, data concerning the use of heterogeneous catalysts (e. g. Nafion and Amberlist based catalysts, montmorillonites containing exchanged metal ions), whose main-merits are the simple separation of the solid catalyst from the reaction mass, the absence of washing water containing the dead catalyst, and the high purity of α-tocopherol (>90 %) were published [Appl. Catal. A: General, 280 (2005) 55; Appl. Catal. A: General, 220 (2001) 51; J. Catal., 182 (1999) 282; Adv. Synth. Catal., 347 (2005) 220; Appl. Catal. A: General, 275 (2004) 247; Catal. Lett., 100 (2005) 101; J. Catal., 176 (1998) 260; Ind. Eng. Chem. Res. 39 (2000) 4487; DE-A 2 743 920, Bull. Chem. Soc. Jpn. 69 (1996) 137; U.S. Pat. No. 3,459,773].

The catalytic application of perfluorinated organic compounds (e.g., Nafion polymer) and lanthanide triflates is a consequence of the great development of the organic fluorine chemistry during the last years [Prog. Solid State Chem., 26 (1998) 97; Chem. Rev., 102 (2002) 2227; Chem. Rev., 103 (2003) 4307; Chem. Commun., (2000) 1695]. Recently, some earth triflate based catalysts were described as a cleaner alternative to the use of metal chlorides as Lewis acid and versatile catalysts [Catal. Today., 121 (2007) 65; U. S. Pat. No. 5,908,939 of Roche Vitamins Inc.] in the synthesis of α-tocopherol, too. A disadvantage of these processes is that triflates are still very expensive and are not available in sufficient quantities. Nafion systems confront many times with problems connected with the catalytic active phase leaching, and montmorrilonite catalysts require complex solids handling for further use.

The discovering and development of inorganic fluorides is closely related with the progress of organic fluorine chemistry. The manufacture of *dl*-[α]-tocopherol by the reaction of TMHQ with phytyl chloride or isophytol in the presence of boron trifluoride ($BF_3$) or its etherate ($BF_3Et_2O$) is described in U.S. Pat. No. 3,444,123 and 4,634,781. However, boron trifluoride has corrosive properties.

In the last years, many solid inorganic fluorides with promising catalytic properties were synthesized. Such materials display high catalytic activity, for example, in reactions as acylation and benzoylation of anisole, $CCl_2F_2$ dismutation and $C_2Cl_4$ hydrofluorination [J. Fluotine Chem., 125 (2004) 937; J. Mater. Chem., 15 (2005) 588].

The problem underlying the present invention is to provide a method for the synthesis of *dl*-[α]-tocopherol by the acid-catalyzed reaction of 2,3,5-trimethylhydroquinone (TMHQ) with isophytol (IP) in the presence of a catalyst which does not have the disadvantages of previously known procedures.

These and other problems are solved by the subject matter of the attached independent claims. Preferred embodiments may be taken from the attached dependent claims.

More specifically, according to the present invention this problem is solved in a first aspect by a method for synthesizing dl-alpha-tocopherol comprising the steps of

- providing 2,3,5-trimethylhydroquinone;
- providing isophytol;

- providing a solid fluoride catalyst; and
- reacting the 2,3,5-trimethylhydroquinone with the isophytol in the presence of the catalyst.

In an embodiment according to the present invention the solid fluoride catalyst is a metal fluoride catalyst of the formula $MF_{n-x}(OH)_x$, wherein

M is metal of the second or third main group;
n is 2 if M is of the second main group or
n is 3 if M is of the third main group; and
x is any figure between 0 and 2, preferably any figure between 0.01 and 0.5.

In a further embodiment according to the present invention the solid fluoride catalyst is either $MgF_{2-x}(OH)_x$ or $AlF_{3-x}(OH)_x$.

In a still further embodiment according to the present invention the 2,3,5-trimethylhydroquinone is reacted with the isophytol in the presence of the catalyst in a two-phase liquid solvent system.

In a preferred embodiment according to the present invention the 2,3,5-trimethylhydroquinone is reacted with the isophytol in the presence of the catalyst under reflux or in closed batch.

In a more preferred embodiment according to the present invention the 2,3,5-trimethylhydroquinone is reacted with isophytol in the presence of the catalyst under an inert atmosphere or in atmospheric medium.

In an embodiment according to the present invention the 2,3,5-trimethylhydroquinone is reacted with isophytol in the presence of the catalyst at a temperature of about 80 °C to 120 °C, preferably about 100 °C.

In an embodiment according to the present invention the ratio, based on equivalents, of 2,3,5-trimethylhydroquinone to isophytol is about 2:1 to 1:2.

According to the present invention this problem is solved in a second aspect by dl-alpha-tocoferol obtainable by a method according to the first aspect of the present invention.

In an embodiment of the second aspect of the present invention the tocoferol does not require further purification.

According to the present invention this problem is solved in a third aspect by the use of a catalyst as defined in the first aspect of the present invention, for alkylation condensation reactions.

The use of the catalyst as described herein for the synthesis of dl-[a]-tocopherol is advantageous in many regards. The catalyst used does not have a corrosive action, is nontoxic, does not contaminate the environment and catalyzes the desired reaction as selectively as possible and in high yields. Furthermore, the catalyst displays its activity already in really only catalytic amounts, and is readily separable and re-usable. Additionally, the catalytic system is advantageous because of its availability and low costs of raw materials needed for catalyst preparation, very simple working set-up for the catalytic synthesis and reduced number of unit operations for the processes. All these advantages are also reflected in the methods for synthesis as described herein.

The catalysts which are preferably used in connection with the methods of the present invention, are characterized by the fact that they are obtained as nanoscopic metal fluorides. Due to the fact that instead of anhydrous HF aqueous solutions of HF are used for the synthesis, they exhibit a unique combinations of Brønsted- and Lewis acid surface sites which may cause their bifunctional properties and, hence, their unexpected unique catalytic properties for this type of reactions. The manufacture of such catalysts follows in part that described in WO 2006/058794 A1 and in H.A. Prescott et al., J. Mater. Chem., 2005, 15, 4616-4628. However, in contrast to what is described there, in the present invention the hydrofluoric acid is added as aqueous solution containing 40-70% (w/w) to the alcoholic aluminium alkoxide solution or as 60-80% (w/w) aqueous solution to the alcoholic magnesium alkoxide solution, and the respective solid products obtained after removal of the solvents are not heated above 150°C, i.e. not calcined.

The present inventors have surprisingly found that solid fluoride catalysts are suitable for performing condensation and more specifically alkylation-condensation reactions. In a preferred embodiment such solid fluoride catalysts are characterised by having acidic centres with moderate strength acidity.

In an embodiment the term acidic centres as used herein means that the catalyst has Lewis and Brønsted acidic sites. It will be acknowledged that methods for determining the amount of acidic centres exhibited by a catalyst can be determined using techniques well known in the art. Such methods comprise titration, $NH_3$-TPD, TG-DTA, catalytic tests, $^{15}$N- and $^{13}$C-NMR, Py-IR spectroscopy.

In an embodiment the term moderate strength acidity as used herein means that the catalyst has sites with medium strength as compared with other catalysts like $SbF_5$, for example, which represents a very strong Lewis acid. It will be acknowledged that methods for determining the acidic strength of a catalyst can be determined using techniques well known in the art. Such methods comprise titration, $NH_3$-TPD, TG-DTA, catalytic tests and CO-IR spectroscopy.

A standard procedure for the characterization of catalytically active acidic surfaces is the IR spectroscopy of adsorbed probe molecules (J. A. Lercher, C. Grundling, G. EderMirth, Catalysis Today 1996, 27, 353; H. Knözinger, S. Huber, J Chem.Soc. Faraday Trans. 1998, 94, 2047; S. B. Sharma, B. L. Meyers, D. T. Chen, J. Miller, J. A. Dumesic, Applied Catalysis A-General 1993, 102, 253; H. G. Karge, V. Dondur, Journal of Physical Chemistry 1990, 94, 765). Electronic interaction between the probe molecule and active centers at the catalyst's surface changes force constants and gives rise to characteristic band shifts. Carbon monoxide, CO, is a very suitable probe molecule for the characterization of strongly acidic surfaces. The wavenumber of the linear $v$(CO) stretching vibration of molecules, adsorbed at Lewis acidic centers without electronic back donation, is shifted to higher wavenumbers than in gaseous CO (Table 1). The $MgF_{2-x}(OH)_x$ catalysts of the present invention used for the synthesis of alpha-tocopherol show a main band between 2180 and 2200 cm$^{-1}$ due to medium Lewis acidic centers, and the $AlF_{3-x}(OH)_x$ catalysts show a main band between 2180 and 2220 cm$^{-1}$ indicating additional stronger Lewis acid centers. Furthermore, in both types of catalysts Brønsted acidic centers could be detected by their band in the region between 2150 and 2180 cm$^{-1}$. Accordingly, the catalyst of the present invention shows a main band in IR spectroncopy between about 2180 to about 2200 cm$^{-1}$. Additionally the catalysts of the present invention show, in an embodiment a band between about 2150 and 2180 cm$^{-1}$ .

Temperature-programmed desorption of $NH_3$ ($NH_3$-TPD) is another widely used method to assess the total number and strength of acid sites (S. B. Sharma, B. L. Meyers, D. T. Chen, J. Miller, J. A. Dumesic, Applied Catalysis A-General 1993, 102, 253; H. G. Karge, V. Dondur, Journal of Physical Chemistry 1990, 94, 765). The amount of desorbed $NH_3$ gives the total number of acid sites. The desorption temperature gives information on the strength of the acid sites. The maximum desorption temperatures of the $MgF_{2-x}(OH)_x$ catalyst of the present invention were centered at 150 °C and 300 °C. The maximum peak at 150 °C can be assign has the Brønsted acidic centers and the maximum peak at 300 °C can be assign has medium Lewis acidic centers.

Accordingly, the catalyst of the present invention shows maximum desorption temperatures at about 150° C and at about 300° C in temperature-programmed desorption of $NH_3$($NH_3$-TPD).

**Table:** Wavenumbers of the $v$(CO) stretching vibration of differently absorbed CO species.

| species | wavenumber |
| --- | --- |
| gaseous CO | 2143 cm$^{-1}$ |
| physisorbed CO | 2140-2150 cm$^{-1}$ |
| CO chemisorbed at | |
| Brønsted acidic centers | 2150 - 2180 cm$^{-1}$ |
| weak Lewis acidic centers | 2160 - 2180 cm$^{-1}$ |
| medium Lewis acidic centers | 2180 - 2200 cm$^{-1}$ |
| strong Lewis acidic centers | 2200 - 2220 cm$^{-1}$ |
| very strong Lewis acidic centers | > 2220 cm$^{-1}$ |

The both different acid sites (Brønsted/Lewis) can be tuned over a wide range by using varying molar ratios of HF to $H_2O$ during the sol-gel-synthesis of the catalysts.

It is within the skills of a person of the art to generate this kind of solid fluoride catalysts. More specifically, the techniques required for such purpose comprise a special sol-gel synthesis for metal fluorides as, e.g. described in [J. Krishna Murthy, Udo Groß, Stephan Rüdiger, Erhard Kemnitz, John M. Winfield, Journal of Solid State Chemistry 179 (2006) 739-746]. The general approach to such "bi-acidic" (Lewis/Brønsted) catalysts is the selective solvolysis of the original M-OR bond by either HF which results in a M-F-bond formation and Lewis acid sites or by $H_2O$ which results in a M-OH-bond formation representing a Bronsted acid site. The variation of the HF/$H_2O$ molar ratio results in a variation of Lewis to Brønsted-surface acid sites.

Since the general principal of adjusting the "bi-acidic" properties of these catalysts is the introduction of fluorine and oxygen in the solid, thus creating Lewis and Brønsted acidity, any synthesis procedure resulting in nanoscopic metal fluorides with a certain content of both, fluorine and oxygen, is applicable for the synthesis of these catalysts. That means, alternately to the most effective synthesis route described above also the sol-gel fluorination with anhydrous HF can be performed using understoichiometric amounts of HF but consequent post thermal treatment which results in the formation of M-O-bonds as a result of metal alkoxide decomposition. The manufacture of such catalysts follows in part that described in WO 2006/058794 A1 and in H.A. Prescott et al., J. Mater. Chem., 2005, 15, 4616-4628.

In an embodiment the solid fluoride catalyst is a sol-gel catalyst.

In another embodiment the solid fluoride catalyst has an active surface of about 100 to 400 m$^2$/g, preferably about 200 to 300 m$^2$/g and more preferably about 250 to 300 m$^2$/g. Methods to determine the active surface are known to the person skilled in the art and include, but are not limited to the BET method as described in [S. Brunauer, P. H. Emmett, E. Teller, J. Am. Chem. Soc., 60 (1938) 309]. Additionally, the catalyst is characterised by a narrow pore size distribution in the mesoporous range with an average diameter of 20-100 Å, preferentially between 20 and 50 A.

In another embodiment the catalyst to be used in accordance with the present invention shows two desorption temperatures as determined by NH$_3$-TPD measurement. The maximum desorption temperatures were centered at 150 °C and 300 °C.

In another embodiment the total amount of acidic centres is about 0.02 to 0.6 mmol/g and more preferably about 0.15 to 0.25 mmol/g.

The catalyst is in a preferred embodiment a metal solid fluoride catalyst which preferably exhibits the characteristics of having few acidic centres and/or having moderate strong activity. The metal component of the catalyst is preferably selected from the second or the third main group of the periodic system. Accordingly, the catalyst used in accordance with the present invention has the following general formula:

$$MF_{n-x}(OH)_x \, ,$$

wherein M is the metal component of the catalyst;

n is 2 if the metal component of the catalyst is selected from the second main group, and
n is 3 if the metal component of the catalyst is selected from the third main group; and
x is any figure between 0 and 2.0, preferentially any figure between 0.01 and 0.5.

Several metals like Zn, Be, Ni, Fe and V, can be used as the metal component of the catalyst, however, most preferably the metal component of the catalyst is Mg or Al. Accordingly, the solid fluoride catalyst is in an embodiment MgF$_{2-x}$(OH)$_x$ or AlF$_{3-x}$(OH)$_x$, preferably having one, several or all of the characteristics of the solid fluoride catalyst described herein.

It will be acknowledged that the sequence of adding or mixing the reactants and the catalyst with each other can be performed in any way. It will also be acknowledged that the use of different solvents or combinations of solvents will or may have an impact on the sequence with which the reactants and the catalyst are added to the reaction system in which the method according to the present invention is practiced and in which the reaction is performed.

In an embodiment the reactions according to the present invention are performed in a two-phase liquid solvent. The two-phase liquid solvent is also referred to herein as the two-phase liquid solvent system in a synonymous manner. Alternatively, the method according to the present invention may be carried out using a one-phase liquid solvent system. Preferably, in such one-phase liquid system the reactant and the catalyst are contained in a single liquid phase. Criteria for the selection of single phase solvents are that both TMHQ and IP are at least slightly soluble at reaction temperature, and that the boiling point is high enough to ensure reasonable reaction temperature under reflux conditions or not too high vapour pressure at reasonable reaction temperatures under closed vial conditions of reaction.

Such single liquid phase is formed in an embodiment by aliphatic and cyclic ketones, including but not limited to isobutyl methyl ketone, and diethyl ketone; aliphatic and cyclic esters, including but not limited to ethyl acetate, and isopropyl acetate; aromatic hydrocarbons, including but not limited to toluene and xylene; aliphatic hydrocarbons, including but not limited to pentane, hexane, and heptane.

The two-phase liquid solvent preferably comprises a first and a second liquid phase. The first liquid phase comprises or is formed by a nonpolar solvent; the second liquid phase comprises or is formed by an aprotic polar solvent. In connection with the method for synthesising dl-[α]-tocopherol in accordance with the present invention, the each of two phases harbour one of the two reactants. More specifically, the aprotic polar phase contains the trimethylhydroquinone, and the nonpolar phase contains the isophytol. The catalyst is preferably contained in or added to the aproptic polar solvent. The reaction products and more specifically dl-[α]-tocopherol, will transfer into the nonpolar solvent. If the first and second liquid solvents are immiscible, a separation from the reaction or from the reaction vessel for further down-stream processing can be advantageously performed.

When using a two-phase liquid solvent system, preferably the starting materials each contained in a solvent, preferably the trimethylhydroquinone in the aprotic polar solvent and the isophytol in the nonpolar solvent, are added to each other and then the catalyst is added, or, alternatively, the catalyst is added to the aprotic polar solvent containing or not yet containing the trimethylhydroquinone, and then the aprotic polar solvent is added to the nonpolar solvent either already containing or not yet containing the isophytol.

With regard to this mode of action, it is within the skills of the person of the art to choose appropriate first and second solvents. Criteria for the selection of a nonpolar solvent are a good solubility for isophytol together with a boiling point which is high enough to ensure reasonable reaction temperature under reflux conditions or not too high vapour pressure at reasonable reaction temperatures under closed vial conditions of reaction.

In an embodiment the nonpolar solvent is selected from the group comprising pentane, hexane, heptane, octane, benzene, toluene, and xylene. Criteria for the selection of the aprotic, i.e. no OH-groups containing polar solvent are a good solubility for TMHQ, and a boiling point similar or higher than that of the nonpolar solvent.

In an embodiment the aprotic polar solvent is selected from the group comprising propylene carbonate, dimethyl carbonate, diethyl carbonate, dipropyl carbonate, ethylene carbonate.

The reaction of 2,3,5-trimethylhydroquinone with isophytol in the presence of the catalyst, preferably in a two-phase liquid solvent system, can be performed under an inert atmosphere or in air. In an embodiment the inert atmosphere is formed by nitrogen and argon, respectively. When the reaction is performed in air, the reaction is performed under an atmosphere essentially suitable to allow human beings to breath. In connection with performing the method according to the present application in or under an air atmosphere, the present inventors found again that they may depart from the respective general understanding of the prior art. For example, it is known from a long time that traces of oxygen in the reaction system can led to quinone compounds as a byproduct decreasing the tocopherol yield [J. Am. Chem. Soc. 85 (1963) 239; J. Chem. Soc. (1965) 5060; Org. Lett. 3 (2001) 3875; Arkivoc 2003, 101]. For this reason the synthesis of tocopherol claims special precautions, e.g., working in inert protective atmosphere [Appl. Catal. A: General, 275 (2004) 247]. Contrarily, in the present work no quinone was detected even if reactions were conducted in air, without any special precautions. The only detected byproduct formed in small amounts, was phytadiene.

In another embodiment, the method according to the present invention is carried out either under reflux or in a closed batch. Both performing the reaction of 2,3,5-trimethylhydroquinone with isophytol in the presence of the catalyst, preferably in a two-phase liquid solvent system, under reflux or in a closed batch results in a closed system which does not allow any exchange of components of the reaction and reaction system with the environment. Under such conditions the atmosphere such as an inert atmosphere or air is essentially maintained except for potential changes of such atmosphere by the reactants and reaction products, respectively, of the reaction and the method according to the present invention, respectively.

In an embodiment, water which may be generated during the reaction of the reactants will be kept in the reaction vessel in which the method according to the present invention is performed. This is realized in those embodiments where the reaction is performed in a closed reaction system such as a closed batch. A closed reaction system as preferably used herein is a reaction system which, at least, does not allow the exchange of material with its environment; however, in one embodiment, the system allows the transfer of energy. As further detailed in the example part, the present inventors have surprisingly found that there is no need for removing the water formed in the progress of the reaction, from the system. Rather than affecting the reaction, the presence of water promotes the general process of condensation. Because of this, performing the method of the present inventions under conditions which promotes the presence of water such as be using a closed reaction system or a closed batch, is clearly and surprisingly advantageous.

In an embodiment of the method according to the present invention, the reaction is carried out at a temperature from about 80 °C to about 120°C. A preferred temperature to react the starting materials in the presence of the catalyst is 100 °C. It will be understood by a person skilled in the art that, in principle, the reaction may be performed at a different temperature. However, without wishing to be bound by any theory, given the starting materials and end-product in case the method according to the present invention is a method for synthesising dl-alpha-tocopherol starting from 2,3,5-trimethylhydroquinone and isophytol, the indicated temperature range is advantageous. However, it will be understood that the catalyst which is used in accordance with the present invention, can be used at a significantly higher temperature.

It will be acknowledged that the reaction time will depend on quite a number of reaction parameters including but not limited to the kind of catalyst used, temperature, concentration of the starting materials, the kind of reaction system used, such as closed batch versus open batch, reaction under nitrogen or under air, intended yield. Preferably the reaction time of 2,3,5-trimethylhydroquinone with the isophytol in the presence of the catalyst ranges from about 30 min to about 720 min.

In an embodiment, the ratio of 2,3,5-trimethylhydroquinone to isophytol in the reaction is, at least initially, i.e. at the beginning of the reaction of the 2,3,5-trimethylhydroquinone (TMHQ) with the isophytol (IP) in the presence of the catalyst, is from about 2:1 or 1:1 to about 1:2. It will be acknowledged that the ratio is either a ratio based on equivalents or a molar ratio. Besides temperature and pressure, the concentration of the reactants is the third parameter that acts upon the reaction equilibrium. Increasing the concentration of IP the reaction rate increases but the dehydration of IP to phytadiene is also favoured leading to the decreases of yield to main product. Increasing the amount of TMHQ this side reaction is disfavoured. Practically there is the same effect as working by adding IP dropwise to the reaction mixture in reflux conditions, as literature claims.

It is within the present invention that the method and catalysts, respectively, described herein are applicable to Friedls-Crafts reactions and more specifically alkylation-condensation reactions with the synthesis of dl-[α]-tocopherol as disclosed herein being one example therefore.

The present invention is now further illustrated by the following figures and example from which further features, embodiments and advantages may be taken.

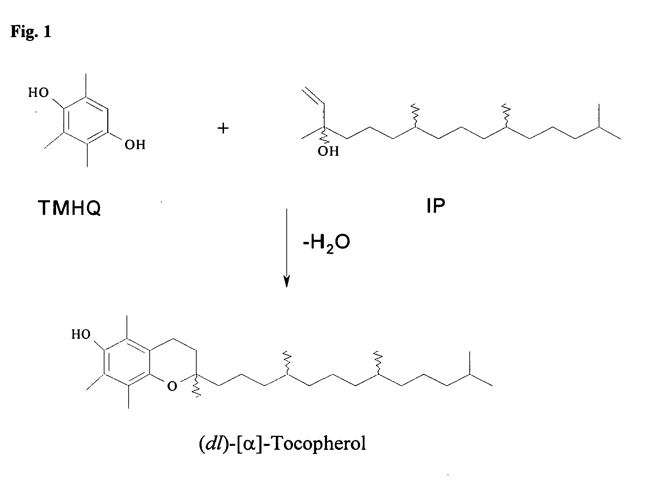

Fig. 1    shows the synthesis of (all-rac)-[α]-tocopherol 4 by the condensation of TMHQ 1 and IP 2.

**Example 1: General procedure for the synthesis of an $MF_{n-x}(OH)_x$ catalysts**

To an alcoholic solution or suspension of the metal alkoxide is at about room temperature under stirring added an 0.8 to 1.3 fold stoichiometric amount of aqueous hydrofluoric acid, the concentration of which is between 40 and 80% (w/w), in case of magnesium alkoxide preferably 60-80% and in case of aluminium alkoxide preferably 40-70%. After an ageing time of 1 to 12 hours all volatiles are removed under vacuum and slowly increased temperature. The final temperature should not exceed 150°C. The as prepared metal fluoride can directly be used as catalyst.
The synthesis procedure is more detailed in the following Examples 1 and 2.

**Example 2: Preparation of magnesium oxide fluoride based catalyst $MgF_{2-x}(OH)_x$**

1.56 g (64 mmol) magnesium turnings are reacted with 50 ml dry methanol at room temperature for about 12 hours followed by 3 hour reflux. To the obtained magnesium methylate solution 150 mmol of an about 70 % hydrofluoric acid are added at room temperature under stirring whereby a viscous sol is formed. After an ageing time of up to 12 hours all volatile components of the sol are removed under vacuum first at room temperature followed by heating up to 70 °C for 5 hours yielding a white solid product.
The thus obtained catalyst had a specific surface area (BET/$N_2$) of 268 $m^2$/g and a carbon content of 0.4 %. This was determined by nitrogen adsorption - desorption experiments carried out at 77K. The specific surface area was calculated according to the BET method. From $NH_3$-TPD measurements a quantitative determination of surface acid sites was performed.

**Example 3: Preparation of aluminium oxide fluoride based catalyst.**

Commercially available aluminium isopropoxide is dissolved in isopropanol and reacted with stoichiometric amounts of an about 50 % hydrofluoric acid whereby a viscous sol is formed. After ageing and drying as in the preceding example a white solid material is formed, which had a specific surface area of 190 $m^2$/g, and a composition of about 54 % F, 1,6 % C and 2,3 % H.

**General procedure for the synthesis of dl-alpha-tocopherol**

152 mg 2,3,5-trimethylhydroquinone (TMHQ) is reacted with isophytol (IP) in a molar ratio of 1:5 to 5:1, preferably of 2:1, in 5 to 50 ml, preferably 6 ml, of a bi-phasic solvent system comprising an aprotic polar phase, such as propylene carbonate, as solvent for TMHQ and a nonpolar phase, such as heptane, as solvent for IP, in the presence of 5 to 500 mg, preferably 10 to 200 mg, more preferably 50 to 200 mg, of a $MgF_{2-x}(OH)_x$ or $AlF_{3-x}(OH)_x$ catalyst under reflux, whereby the IP can be dropwise added, or preferably in a closed vial at a temperature of 50 to 200°C, preferably at 100-120 °C, over a period of 30 min to 20 hrs, preferably 60 min, under vigorous stirring. The reaction mixture is allowed to cool to room temperature and then the catalyst is separated by filtration or centrifugation, the nonpolar heptane phase containing the product dl-alpha-tocopherol is separated and the heptane removed by distillation yielding the product. Analysis can be done by, e.g., using HPLC or $^{13}$C- and $^1$H-NMR, the $^1$H-NMR ($CDCl_3$) data for (all-rac)-[$\alpha$]-tocopherol are 0.90 ppm (m, 12 H, $CH_3$), 1.0-1.9 ppm (m, 26 H, $CH_3$, $CH_2$, CH), 2.10 and 2.17 ppm (both s, 9 H, $CH_3$-Ar), 2.60 ppm (t, 2 H, $C^4H_2$), 4.3 (s, 1 H, OH). The separated catalyst can be re-used after washing with acetone, drying, and heating under a flow of nitrogen at 100 °C for 6 to 48 hrs, preferably for 12 to 24 hrs.
The synthesis procedure is more detailed in the following Examples 4 to 7.

**Example 4: Synthesis of dl-alpha-tocopherol**

Example 4a

A 150 mL stirred batch equipped with an attached reflux condenser was charged with 10 ml propylene carbonate. 152 mg trimethylhydroquinone (TMHQ, purity > 97 %), 10 ml heptane, 50 mg of $MgF_{2-x}(OH)_x$ and the batch was heated at 100 °C with stirring, 0,4 ml isophytol (IP, purity 98 %)(1 1 molar ratio of TMHQ /IP) were then added dropwise in the course of 30 min to the boiling mixture. The mixture was then allowed to react for 120 min at 100 °C.

Example 4b

A 8 ml stirred glass vial was charged with 3 ml propylene carbonate, 152 mg trimethylhydroquinone (TMHQ, purity > 97 %), 3 ml heptane, 0,4 ml isophytol (IP, purity 98 %) and 50 mg of $MgF_{2-x}(OH)_x$ (Table 2). The mixture was then allowed to react for 60 min at 100 °C under vigorous stirring. In both examples, after completion the reaction, the catalyst was filtering-off, the heptane phase containing tocopherol was separated and the solvent was removed under vacuum. The crude product was analysed by $^1$H-NMR and $^{13}$C-NMR.

The results of both Example 4a and 4b are show in Table 2

Table 2.

| Ex. | Time, (min) | Conversion of IP, (%) | Yield to tocopherol, (%) |
|---|---|---|---|
| 4a | 120 | 98.6 | 36.8 |
| 4b | 60 | 100 | 74.6 |

Example 4b shows the influence of the water formed in synthesis on the catalyst activity and the yield to tocopherol. When used closed glass vial a significant increases of both factors is observed.

**Examples 5 and 6: Further synthesis of dl-alpha-tocopherol**

The procedure of comparative Example 4b was repeated by changing the amount of catalyst (Example 5) or the amount of TMHQ (Example 6). In Example 5a, $MgF_{2-x}(OH)_x$ is replaced with commercial $MgF_2$ (purchased from Merck, purity of 98 %). In Example 6, 0.5 mol IP based on TMHQ means to add in process 304 mg TMHG and 0.4 ml IP. Results obtained from these experiments are given in Table 3.

Table 3

| Ex. | Amount of $MgF_{2-x}(OH)_x$, (mG) | IP mols | Time, (min) | Conversion of IP, (%) | Yield to tochopherol, (%) |
|---|---|---|---|---|---|
| 5a | 50* | 1 | 300 | 0 | 0 |
| 5b | 10 | 1 | 300 | 100 | 26.3 |
| 5c | 50 | 1 | 60 | 100 | 74.6 |
| 5d | 200 | 1 | 60 | 100 | 92.6 |
| 6 | 50 | 0.5 | 60 | 100 | 90.1 |
|  | 50 | 0.5 | 180 | 100 | 100 |

*commercial $MgF_2$ used (ALDRICH, 98%)

**Example 7: Further synthesis of dl-alpha-tocopherol**

The procedure from comparative Example 4b was repeated by changing the catalyst from $MgF_{2-x}(OH)_x$ to $AlF_{3-x}(OH)_x$. In Example 7a $MgF_{2-x}(OH)_x$ is replaced with a pyrochlor aluminium fluoride (ZAAC 628, 162 (2002)) The following results were obtained (Table 4).

Table 4

| Ex. | Amount of $AlF_{3-x}(OH)_x$ (mG) | IP mols | Time, (min) | Conversion of IP, (%) | Yield to tochopherol, (%) |
|---|---|---|---|---|---|
| 7a | 50* | 1 | 240 | 0 | 0 |
| 7b | 10 | 1 | 300 | 100 | 82.6 |
| 7c | 50 | 1 | 720 | 100 | 100 |
| 7d | 200 | 1 | 60 | 100 | 99.8 |
| 7f | 50 | 0.5 | 60 | 100 | 100 |

* pyrochlor aluminium fluoride used

The features of the present invention disclosed in the specification, the claims and/or the drawings may both separately and in any combination thereof be material for realizing the invention in various forms thereof.

**Claims**

1. A method for synthesizing dl-alpha-tocopherol comprising the steps of

- providing 2,3,5-trimethylhydroquinone;
- providing isophytol;
- providing a solid fluoride catalyst; and
- reacting the 2,3,5-trimethylhydroquinone with the isophytol in the presence of the catalyst.

2. The method according to claim 1, wherein the solid fluoride catalyst is a metal fluoride catalyst of the formula $MF_{n-x}(OH)_x$, wherein

M is metal of the second or third main group;
n is 2 if M is of the second main group or
n is 3 if M is of the third main group; and
x is any figure between 0 and 2, preferably any figure between 0.01 and 0.5.

3. The method according to any of claims 1 to 2, wherein the solid fluoride catalyst is either $MgF_{2-x}(OH)_x$ or $AlF_{3-x}(OH)_x$.

4. The method according to any of claims 1 to 3, wherein the 2,3,5-trimethylhydroquinone is reacted with the isophytol in the presence of the catalyst in a two-phase liquid solvent system.

5. The method according any of claims 1 to 4, wherein the 2,3,5-trimethylhydroquinone is reacted with the isophytol in the presence of the catalyst under reflux or in closed batch.

6. The method according to any of claims 1 to 5, wherein the 2,3,5-trimethylhydroquinone is reacted with isophytol in the presence of the catalyst under an inert atmosphere or in atmospheric medium.

7. The method according to any of claims 1 to 6, wherein the 2,3,5-trimethylhydroquinone is reacted with isophytol in the presence of the catalyst at a temperature of about 80 °C to 120 °C, preferably about 100 °C.

8. The method according to any of claims 1 to 7, wherein the ratio, based on equivalents, of 2,3,5-trimethylhydroquinone to isophytol is about 2:1 to 1:2.

9. dl-alpha-tocoferol obtainable by a method according to any of claims 1 to 8.

10. The dl-alpha-tocoferol according to claim 9, whereby the tocoferol does not require further purification.

11. Use of a catalyst as defined in any of claim 1 to 3 for alkylation condensation reactions.

**Fig. 1**

TMHQ   +   IP

-H$_2$O

(dl)-[α]-Tocopherol

**European Patent Office**

## EUROPEAN SEARCH REPORT

Application Number

EP 07 02 0498

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | BONRATH ET AL: "Catalysis in the industrial preparation of vitamins and nutraceuticals" CATALYSIS TODAY, ELSEVIER, vol. 121, no. 1-2, 13 February 2007 (2007-02-13), pages 45-57, XP005886134 ISSN: 0920-5861 | 9,10 | INV. C07D311/72 C01B11/24 C01F5/28 C01F7/50 |
| X | * page 47 - page 51 * | 1 | |
| X,D | LAUFER M C ET AL: "Synthesis of (all-rac)-[alpha]-tocopherol using Nafion resin/silica nanocomposite materials as catalysts" CATALYSIS LETTERS, KLUWER ACADEMIC PUBLISHERS-PLENUM PUBLISHERS, NE, vol. 100, no. 1-2, 1 March 2005 (2005-03-01), pages 101-109, XP019275103 ISSN: 1572-879X * the whole document * | 1-8 | |
| X,D | SCHAGER F ET AL: "Synthesis ofD,L-alpha-Tocopherol Using Strong Solid Acids as Catalysts" JOURNAL OF CATALYSIS, ACADEMIC PRESS, DULUTH, MN, US, vol. 182, no. 1, 15 February 1999 (1999-02-15), pages 282-284, XP004443242 ISSN: 0021-9517 * page 282 * | 1-8 | TECHNICAL FIELDS SEARCHED (IPC) C07D C01B C01F |
| | -/-- | | |

~~The present search report has been drawn up for all claims~~

| Place of search | Date of completion of the search | Examiner |
|---|---|---|
| Munich | 28 February 2008 | Fazzi, Raffaella |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 
& : member of the same patent family, corresponding document

EPO FORM 1503 03.82 (P04C01)

**European Patent Office**

## EUROPEAN SEARCH REPORT

Application Number

EP 07 02 0498

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WANG H ET AL: "Catalytic performance of Nafion/SiO2 nanocomposites for the synthesis of alpha-tocopherol" APPLIED CATALYSIS A: GENERAL, ELSEVIER SCIENCE, AMSTERDAM, NL, vol. 275, no. 1-2, 8 November 2004 (2004-11-08), pages 247-255, XP004578977 ISSN: 0926-860X * page 248 * | 1-8 | |
| X,D | US 5 908 939 A (BAAK MARCEL [CH] ET AL) 1 June 1999 (1999-06-01) * column 3 - column 4 * | 1-8 | |
| X | EP 0 658 552 A (EISAI CO LTD [JP]) 21 June 1995 (1995-06-21) * examples * | 1-8 | |

TECHNICAL FIELDS
SEARCHED    (IPC)

The present search report has been drawn up for all claims

| Place of search | Date of completion of the search | Examiner |
|---|---|---|
| Munich | 28 February 2008 | Fazzi, Raffaella |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

                                                     

& : member of the same patent family, corresponding
    document

EPO FORM 1503 03.82 (P04C01)

European Patent
Office

Application Number

EP 07 02 0498

## CLAIMS INCURRING FEES

The present European patent application comprised at the time of filing more than ten claims.

☐ Only part of the claims have been paid within the prescribed time limit. The present European search report has been drawn up for the first ten claims and for those claims for which claims fees have been paid, namely claim(s):

☐ No claims fees have been paid within the prescribed time limit. The present European search report has been drawn up for the first ten claims.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

1-10

☐ The present supplementary European search report has been drawn up for those parts of the European patent application which relate to the invention first mentioned in the claims (Rule 164 (1) EPC).

**European Patent Office** | **LACK OF UNITY OF INVENTION** | Application Number

**SHEET B** | EP 07 02 0498

The Search Division considers that the present European patent application does not comply with the requirements of unity of invention and relates to several inventions or groups of inventions, namely:

1. claims: 1-10

   Present claims 1-8 describe a process for the preparation of dl-alpha-tocopherol by using a solid fluoride catalyst; claims 9-10 relate to the dl-alpha-tocopherol obtainable by said process.
   ---

2. claim: 11

   Claim 11 discloses the use of a catalyst of formula MF(n-x)(OH)x  for alkylation condensation reactions.
   ---

## EP 2 050 743 A1

**ANNEX TO THE EUROPEAN SEARCH REPORT
ON EUROPEAN PATENT APPLICATION NO.**

EP 07 02 0498

This annex lists the patent family members relating to the patent documents cited in the above-mentioned European search report.
The members are as contained in the European Patent Office EDP file on
The European Patent Office is in no way liable for these particulars which are merely given for the purpose of information.

28-02-2008

| Patent document cited in search report | | Publication date | Patent family member(s) | | Publication date |
|---|---|---|---|---|---|
| US 5908939 | A | 01-06-1999 | CN | 1237163 A | 01-12-1999 |
| | | | DE | 69711669 D1 | 08-05-2002 |
| | | | DE | 69711669 T2 | 02-10-2002 |
| | | | DK | 937055 T3 | 29-07-2002 |
| | | | WO | 9821197 A2 | 22-05-1998 |
| | | | ES | 2173500 T3 | 16-10-2002 |
| | | | JP | 2001504111 T | 27-03-2001 |
| EP 0658552 | A | 21-06-1995 | CA | 2137481 A1 | 15-06-1995 |
| | | | CN | 1108254 A | 13-09-1995 |
| | | | DE | 69417527 D1 | 06-05-1999 |
| | | | DE | 69417527 T2 | 26-08-1999 |
| | | | JP | 3848380 B2 | 22-11-2006 |
| | | | JP | 7224054 A | 22-08-1995 |
| | | | US | 5532387 A | 02-07-1996 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2411969 A, Hoffmann-La Roche
- US 4239691 A
- DE 3203487
- US 6005122 A
- DE 2743920
- DE 2743920 A
- US 3459773 A
- US 5908939 A
- US 3444123 A
- US 4634781 A
- WO 2006058794 A1

**Non-patent literature cited in the description**

- *Catal. Today,* 2007, vol. 121, 45
- *Mol. Nutr. Food Res.,* 2005, vol. 49, 7
- *Biomedicine & Pharmacotherapy,* 2005, vol. 59, 380
- *Nutrition Research,* 2005, vol. 25, 877
- *Chimica,* 1996, vol. 50, 563
- *Appl. Catal. A: General,* 2005, vol. 280, 55
- *Appl. Catal. A: General,* 2001, vol. 220, 51
- *J. Catal.,* 1999, vol. 182, 282
- *Adv. Synth. Catal.,* 2005, vol. 347, 220
- *Appl. Catal. A: General,* 2004, vol. 275, 247
- *Catal. Lett.,* 2005, vol. 100, 101
- *J. Catal.,* 1998, vol. 176, 260
- *Ind. Eng. Chem. Res.,* 2000, vol. 39, 4487
- *Bull. Chem. Soc. Jpn.,* 1996, vol. 69, 137
- *Prog. Solid State Chem.,* 1998, vol. 26, 97
- *Chem. Rev.,* 2002, vol. 102, 2227
- *Chem. Rev.,* 2003, vol. 103, 4307
- *Chem. Commun.,* 2000, 1695
- *Catal. Today.,* 2007, vol. 121, 65
- *J. Fluotine Chem.,* 2004, vol. 125, 937
- *J. Mater. Chem.,* 2005, vol. 15, 588
- **H.A. PRESCOTT et al.** *J. Mater. Chem.,* 2005, vol. 15, 4616-4628
- **J. A. LERCHER ; C. GRUNDLING ; G. EDER-MIRTH.** *Catalysis Today,* 1996, vol. 27, 353
- **H. KNÖZINGER ; S. HUBER.** *J Chem.Soc. Faraday Trans.,* 1998, vol. 94, 2047
- **S. B. SHARMA ; B. L. MEYERS ; D. T. CHEN ; J. MILLER ; J. A. DUMESIC.** *Applied Catalysis A-General,* 1993, vol. 102, 253
- **H. G. KARGE ; V. DONDUR.** *Journal of Physical Chemistry,* 1990, vol. 94, 765
- **J. KRISHNA MURTHY ; UDO GROß ; STEPHAN RÜDIGER ; ERHARD KEMNITZ ; JOHN M. WINFIELD.** *Journal of Solid State Chemistry,* 2006, vol. 179, 739-746
- **S. BRUNAUER ; P. H. EMMETT ; E. TELLER.** *J. Am. Chem. Soc.,* 1938, vol. 60, 309
- *J. Am. Chem. Soc.,* 1963, vol. 85, 239
- *J. Chem. Soc.,* 1965, 5060
- *Org. Lett.,* 2001, vol. 3, 3875
- *Arkivoc,* 2003, 101
- *ZAAC,* 2002, vol. 628, 162